(19) **Europäisches Patentamt / European Patent Office / Office européen des brevets**

(11) **EP 1 859 866 A1**

(12) **EUROPEAN PATENT APPLICATION**

(43) Date of publication:
**28.11.2007 Bulletin 2007/48**

(51) Int Cl.:
**B01L 3/00** *(2006.01)* **C12N 5/00** *(2006.01)*

(21) Application number: **07250717.1**

(22) Date of filing: **21.02.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **23.05.2006 US 438686**

(71) Applicant: **Genetix Limited**
**New Milton,**
**Hampshire BH25 5NN (GB)**

(72) Inventor: **Jiang, Yonggang**
**New Milton**
**Hampshire BH25 5GG (GB)**

(74) Representative: **Haines, Miles John L.S.**
**D Young & Co**
**120 Holborn**
**London EC1N 2DY (GB)**

(54) **Well plate**

(57) A well plate (20) of non-unitary construction. The well plate (20) is constructed from a first part (26) of interconnected tubes that define the side walls of each well (22) and a second part (28) defining the well bases. The hydrophobicity of the first part (26) is selected to suppress meniscus formation in the wells (22), thereby assisting optical characterization of samples in the wells (22), in particular by phase contrast microscopy. This can be achieved by using a natural cycloolefin copolymer (COP) which is not subjected to the usual plasma treatment to improve its wetting. The second part (28) is formed of a single transparent sheet of hydrophilic material, such as a COP, which is bonded to the first part (26), for example ultrasonically.

Fig. 4

EP 1 859 866 A1

**Description**

## BACKGROUND OF THE INVENTION

The present invention generally relates to well plates.

Well plates are used widely for handling liquid samples in chemical and biological studies in fields such as gene sequencing, combinatorial chemistry, drug discovery and proteomics. Well plates can be used in automated and integrated systems and methods for rapidly identifying chemicals with biological activity in liquid samples, particularly automated screening of low volume samples for new medicines, agrochemicals, or cosmetics.

The samples, e.g. cells or cell colonies, held in liquid in the well plates often need to be analyzed optically, which may be in microscope imaging, but commonly involves some form of spectroscopic measurements, such as fluorescence measurements of chemical or biological samples. For example, samples are often fluorescently tagged.

A number of multi-well well plate platforms are commercially available from different vendors for culturing cells or performing chemical or cellular assays. These feature arrays of wells that conform to an industry standard grid which is based on a square gird of 4.5 mm. The well spacings may exactly follow this grid, or follow integer multiples or whole fractions thereof. Currently, the most common formats are 96 or 384 wells, but other numbers of wells are also known, such as 864 or 1536, with well plates with larger numbers of wells and consequently smaller liquid capacities becoming more prevalent as automation improves and combinatorial approaches develop. Well plates with lower numbers of wells such as 6, 24 or 48, are also available as are single-well well plates which are effectively Petri dishes with a convenient outer form compatible with industry standard robotic well plate handling and tracking systems. The industry standard outer dimensions are unfortunately not closely adhered to, with the following dimension ranges being used: length 127-128 mm and width 84-85 mm. The depths are even less standardized with depth dimensions in the range 10-50 mm being available, although depths of 14-15 mm may be termed standard. A convenient summary of different vendors is provided in Table I of reference [1].

While many of these well plates offer the desirable features of biocompatibility, ease of manufacture and substantial structural integrity, there is another problem that arises when using well plates.

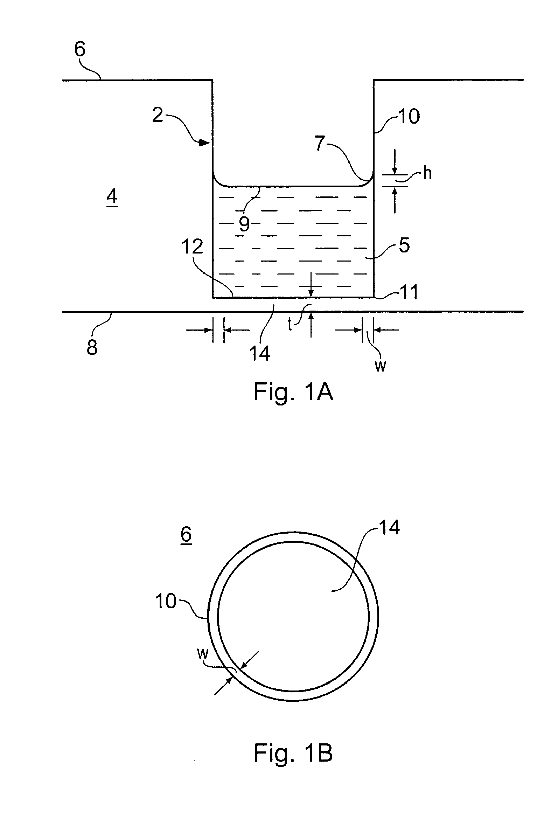

Figures 1A and 1B of the accompanying drawings schematically illustrate in side section and plan view a single well 2 of a standard well plate 4 that is partially filled with a liquid 5.

The well plate 4 is formed in a plastics material, most commonly polystyrene (PS), but also propylene (PP) for some applications, as a unitary molded construction in which an array of wells 2 conforming to an industry standard square grid is formed by cylindrical depressions extending down from a top face 6 towards a bottom face 8.

Each well 2 comprises a cylindrical side wall 10 and a base 12 which is shown as a flat base, since this is the preferred shape for well plates that will be used for optical analysis. A window 14 of thickness 't' that is transparent to a range of desired wavelengths used in the intended optical analysis methods, typically in the visible range, is thus formed between the well plate base 12 and the adjacent part of the bottom face 8 of the well plate. The window 14 may be used for optical access to a sample either in respect of optical excitation or optical collection, or both.

While Figures 1A and 1B illustrate a well plate of unitary construction, well plates of two part construction are also known in the art [13, 14].

It is noted that for fluorescence measurements a known problem with well plates made of standard material is the relatively high amount of background fluorescence inherent in the material. To reduce this problem, special well plates are available that are made of a low-fluorescence material [1].

Because of surface tension effects and the hydrophilic well plate surfaces required for cell culturing, the liquid's liquid-air interface 9 forms a concave meniscus 7 of height 'h' and width 'w' adjacent the side wall 10, as schematically illustrated. The meniscus dimensions are determined by the balancing of forces between the cohesion of the liquid itself and the adhesion between the liquid and the solid surface, in this case the well wall 10. Cohesion refers to the intermolecular attractive force of the liquid molecules. Adhesion refers to the attractive force between the liquid molecules and the surface. It is noted that the hydrophilic nature of the well plate is a result of its surface treatment, typically by a plasma process, which is carried out to improve wettability and thereby allow cells to adhere on the surfaces.

While traditionally unproblematic, as assay volumes become smaller, the meniscus formation can cause major difficulties. In particular, for assays with a volume of less than about 200 microliters, e.g. 1-100 microliters, the meniscus can significantly disrupt the optics, thereby impeding well plate applications in such assays.

For example, if phase contrast imaging is used, the path length through the meniscus region is considerably longer than through other parts of the liquid, causing an obscured dark ring to appear when the main central part of the liquid column is in view.

The problem is further compounded, when one is predominantly interested in the region of the sample adjacent the side walls. For example, single cell clones tend to concentrate at the side walls, most especially at the junction 11 between the side wall 10 and base 12. Optical measurements of the growth of such single cell clones are therefore obscured by this edge effect darkness.

Identification of cells growing at the edge of plates is important for clonal selection of mammalian cells, which is a process that is very important for the production of cell lines and monoclonal antibody production. There are also other applications where imaging at the boundary may be especially important in the agrochemical, pharmaceutical, environmental and cosmetic fields in which large numbers of liquid samples containing chemicals are processed.

In addition to the effect that the meniscus has on optical characterization, the fact that meniscus formation is relatively more pronounced in smaller wells has also been associated with problems in conventional cell culturing protocols [2]. In this study it was observed that in 96-well plates significant populations of cells died shortly after refeeding, where the cell death was concentrated in a ring adjacent the side wall where the meniscus was formed. It was noted that although this effect occurs in larger wells (e.g. 24-well plates), it is more significant in smaller well formats where the ratio of side wall circumference to cell area is greater.

## SUMMARY OF THE INVENTION

In one aspect the invention provides a well plate having at least one well, each well being defined by a side wall and a base, wherein the side wall has a surface energy that provides a contact angle of approximately 90 degrees, thereby to inhibit meniscus formation. Contact angle is defined for an aqueous solution contained in the well in an ambient (air) environment. By a contact angle of approximately 90 degrees we mean between any one of 60, 70, 80 or 85 degrees and any one of 95, 100, 110 or 120 degrees. By way of comparison, conventional the plasma treated surfaces of conventional polystyrene well plates have contact angles of around 20 degrees.

With a contact angle of precisely 90 degrees, the meniscus height will be zero. In reality, the benefit of the invention can be gained by sufficiently reducing the meniscus formation so that the undesirable optical and/or biological effects of interest discussed above are substantially reduced or avoided. For example, if the main aim is to ensure that phase contrast microscopy will be able to image across the full width of each well, then it will be sufficient so long as the height of the meniscus is reduced to below about a 1/2 wavelength of the light being used, typically to less than about 200-350 nm for visible microscopy, more preferably to below 1/10 wavelength, e.g. 40-70 nm.

To make the well plate compatible with robotics and automation equipment, the well plate can have a footprint of a standard 96-well microtiter plate. The well plate itself may be a 96 well plate, or of any desired number, including less than 96, e.g. 48, 24, 6 or 1. However, the invention comes into its own with high density well plates, since through suppression of the meniscus formation, it is possible to miniaturize and increase the throughput through automation equipment by providing well plates with a higher density of smaller wells within the standard footprint, e.g. ·384, 864 or 1536 wells, or still higher density formats such as greater than 3,000 wells per well plate.

Furthermore, small volume wells may be necessary or desirable owing to the costs associated with reagents, some of which are significantly expensive. For example, the wells can have volumes (when filled to the top of the side walls) less than ten, five or three microliters, specifically between 1-10 microliters. It is through provision of the meniscus suppressing side wall surface that this becomes possible without introducing the above-mentioned problems with imaging and cell death.

In some cases it is useful that the base has a surface energy that is lower than the side wall surface energy to promote preferential attachment of cells to the base relative to the side wall. For example, a hydrophobic wall and hydrophilic base.

Moreover, it is preferable that at least the base and optionally also the side wall is made of transparent material for optical characterization and also general viewing. By transparent we mean transmissive of optical radiation at wavelength ranges of interest, typically in the visible range of 400 to 700 nm, but optionally including also the near infra-red, e.g. up to 800 or 900 nm and ultra-violet, e.g. down to 300 nm, or sub-ranges thereof, e.g. ranges in the visible defined by particular colors such as red, green or blue.

At least the base and optionally also the side wall is made of a low-fluorescence material having less than 80% of the fluorescence per unit volume of polystyrene. In particular, any of the materials proposed by Coassin et al [1] can be used, principally materials comprising a cycloolefin polymer, in particular a cycloolefin (co-)polymer (COP) as are available from several vendors such as Japan Synthetic Rubber, Mitsui Chemical Corporation, Ticona and Zeon Corporation [9, 11].

Whereas the side wall have a contact angle of approximately 90 degrees, the base is preferably hydrophilic, i.e. has a surface energy that provides a contact angle of between any one of 2, 4, 10, 15, 20 or 30 degrees and any one of 25, 30, 35 or 50 degrees, in particular between 15 and 25 degrees. The contact angle is defined for an aqueous solution (water) contained in the well in an ambient (air) environment for the usual samples of interest.

The side wall can be made of a material that naturally has a contact angle of approximately 90 degrees (80-100 degrees), such as polyethylene (PE) or polystyrene (PS), polypropylene (PP) or some COPs. By naturally, we mean that the material exhibits this property without any surface treatment for improving wettability such as plasma treatment. Alternatively, the side wall material can be made of a material that has a strong natural hydrophobicity (e.g. some COPs) that has been treated to increase its surface energy and thereby reduce the contact angle to approximately 90 degrees.

Furthermore, the side wall material can be made of a material that has a strong natural hydrophilicity that has been treated to decrease its surface energy and thereby increase the contact angle to approximately 90 degrees. The side wall material can be one of the group: cycloolefin polymer (such as Zeonor), polyethylene, polystyrene, polypropylene, and polymethyl methacrylate.

In another aspect the invention provides a well plate of non-unitary construction having at least one well, each well being defined by a side wall and a base, wherein the well plate is constructed from a first part defining the side wall of each well and a second part defining the base of each wall. A two-part construction is particularly favored, although the well plate could have more parts, such as three or more. More especially, in a preferred form, the first part comprises multiple side walls interconnected laterally by a web of the same material, and the second part comprises a generally flat bottom including areas defining the well bases. The second part may have the particularly simple form of a flat sheet of uniform thickness, which may be transparent and optionally also of low-fluorescence material. Many COPs are transparent for example. The first part may be opaque, for example black to inhibit spurious reflections during optical characterization, and may also benefit from being made of low-fluorescence material.

Separating out the side wall and bases into distinct physical components allows complete design independence over the properties of the side walls and bases.

In particular this allows design freedom to select the surface energy of the side wall needed to suppress meniscus formation independently of the properties required for the base which may be one or both of selection of a base surface energy to promote preferential adherent cell colony growth on the base rather than the side wall and selection of required optical properties, such as transparency and low-fluorescence.

The first part preferably has a first surface energy for the side wall of each well. The second part preferably has a second surface energy for the base of each well. In some embodiments, the second surface energy for the base of each well is lower than the first surface energy to promote preferential attachment of cells to the base relative to the side wall. In other embodiments, the second surface energy for the base of each well is higher than the first surface energy to promote cell growth of cells adhered to the base relative to cells adhered to the side wall. The choice will depend on the intended use of the well plates. The second part may be transparent.

A highly significant advantage of the non-unitary construction is that the well bases can be made very thin and with good optical properties while still having sufficient structural integrity. In particular it is possible to make a thin, good optical quality transparent second part compared with what can be achieved in the conventional unitary construction based on molding.

A thin base is important optically, since the inherent background fluorescence of the well plate material often defines the noise floor for fluorescence measurements, whether the well plate is made of conventional transparent plastics material, low-fluorescence plastics material or glass. Since the background fluorescence scales per unit area of the base it scales linearly with base thickness. To put it most simply, *ceteris paribus,* if the base is half the thickness it will have half the background fluorescence.

Moreover, by making the well bases from a separate part from the well walls and forming the wells by sealingly abutting the walls and bases, the optical integrity of the base is maintained across its full lateral extent and specifically does not deteriorate in the edge region which may be an area of particular interest, for example when cells preferentially adhere and grow at the wall-base junction.

The specific combination of good optical quality of the base, and meniscus suppression provided by the side walls can be used to provide high or low well-density well plates that offer excellent performance characteristics in terms of observation properties at the boundary between the bottom and edge of the wells when containing liquid making it possible to observe cells more easily.

The base material may be made of a variety of materials, preferably transparent materials, such as transparent polycarbonate (PC), transparent polymethyl methacrylate (PMMA), COP, or glass.

In a further aspect the invention provides a method of manufacturing a well plate having at least one well, each well being defined by a side wall and a base, the method comprising: providing a first part defining the side wall of each well; providing a second part defining the base of each wall; and connecting the first and second parts so that the base of each well forms a water-tight seal with its associated side wall.

The first part may be made of a material that naturally has the desired surface energy to provide a contact angle of approximately 90 degrees (between any one of 80 or 85 degrees and any one of 95 and 100 degrees). Alternatively, the first part may be treated to modify its surface energy to provide the desired contact angle of approximately 90 degrees for a water-air interface abutting the side wall. For example, the first part can be made of a material that is naturally strongly hydrophobic with a contact angle of greater than 90 degrees (e.g. some COPs) and the treatment used to increase its surface energy until the contact angle is reduced to the desired value of approximately 90 degrees.

The treatment may be using a plasma process. For example, plasma treatment may occur in an oxygen atmosphere. However, the surface treatment could be achieved by other known processes, such as UV exposure [10, 11], flame treatment [11] or corona treatment [11], grafting [11] and plasma polymerization [11]. Suitable processes may be taken not only from the field of biological sample containers for cell culturing, but also from micro-fluidics.

The second part may also be treated to modify its surface energy to make it lower than that of the first part to promote preferential attachment of cells to the base relative to the side wall. In some instances, the reverse may be desired, i.e. the second material provides a second surface energy for the base of each well which is higher than the first surface energy. In some cases, this promotes cell growth of cells adhered to the base relative to cells adhered to the side wall.

It will be understood that the first part may have a unitary construction, or may itself be formed of multiple parts. Similarly, the second part may have a unitary construction, or may itself be formed of multiple parts.

Further aspects of the invention relate to use of the well plates of the invention for assaying and other applications.

A further aspect of the present invention is a method for detecting the presence of the cells in a sample contained in a well plate. The method can be based on fluorescence, so that light emitted from a sample within a well of a well plate is measured. The amount of fluorescence emitted from the well is indicative of a reaction within the well.

Another aspect of the present invention is a method for identifying a modulator of a biological process such as cell growth or target in a sample contained in a well of a well plate. The method can use fluorescence, so that light emitted from the sample is detected. In practicing the method a biological process or target is contacted with a test chemical. A substrate, a cell or proteins secreted from a cell and contained within a well of a well plate, can be excited with radiation of a first wavelength. Radiation of a second wavelength emitted from the sample is measured and can be indicative of the presence of a modulator within the sample. This method can be used to identify useful compounds; thus the present invention includes compounds identified by these methods.

A further aspect of the present invention is a method to identify a therapeutic by contacting a test chemical with the cell, biological process or target. This mixture, contained within a well of a well plate of the present invention, is excited with radiation of a first wavelength. Radiation of a second wavelength emitted from the sample is measured and can be indicative of the presence of a therapeutic within the sample. This method can be used to identify therapeutics; thus the present invention includes therapeutics identified by this method. A therapeutic identified using this method can be provided in a pharmaceutically acceptable carrier.

A further aspect of the present invention is a method of testing a therapeutic for therapeutic activity and toxicology. The method identifies a therapeutic using a method of the present invention. The identified therapeutic is then monitored for toxicity and efficacy on cells growing in the well plates described here.

A still further aspect of the invention is a method of cell counting and/or confluence detection, in which cells are cultured in a well plate of non-unitary construction having at least one well, each well being defined by a side wall and a base, wherein the well plate is constructed from a first part defining the side wall of each well and a second part defining the base of each wall, wherein the base has a surface energy that is lower than the side wall surface energy to promote preferential attachment of cells to the base relative to the side wall, so that cells substantially grow in a single plane defined by the base. This assists imaging, since all cells will lie in a common focal plane of the imaging apparatus used to capture the images used for image processing to count and/or measure confluence of the cells. Moreover, it also assists counting, since cells will not overlap each other, as viewed in the vertical direction, which makes the image processing more difficult.

Specifically, the invention provides in one aspect a method comprising: providing a well plate having at least one well, each well being defined by a side wall formed by a first part of the well plate and a base formed by a second part of the well plate, wherein for each well the base has a surface energy that is lower than the surface energy of the side wall; and growing cells in aqueous solution in each well, wherein the cells preferentially attach to the base owing to the lower surface energy of the base relative to the side wall. It will be understood that the first part may have a unitary construction, or may itself be formed of multiple parts. Similarly, the second part may have a unitary construction, or may itself be formed of multiple parts.

The well plate may be imaged from above or below to view the cells that have grown. Moreover, images of a plane at or adjacent to the base may be digitally stored and then processed to count the cells and/or determine a degree of confluence of the cells, typically on a well-by-well basis. It will be appreciated that the cells may be individual cells, e.g. mammalian cells, or may form one or more colonies in each well.

## BRIEF DESCRIPTION OF THE DRAWINGS

For a better understanding of the invention and to show how the same may be carried into effect reference is now made by way of example to the accompanying drawings in which:

Figures 1A and 1B schematically illustrate in side section and plan view a single well of a standard well plate that is partially filled with a liquid;

Figure 2 illustrates meniscus formation in a well plate;

Figure 3 schematically illustrates in side section view a single well of a well plate embodying the invention; and

Figure 4 is a perspective exploded view of a well plate embodying the invention.

## DETAILED DESCRIPTION

Before describing a well plate embodying the invention, some relevant general background regarding meniscus formation is summarized.

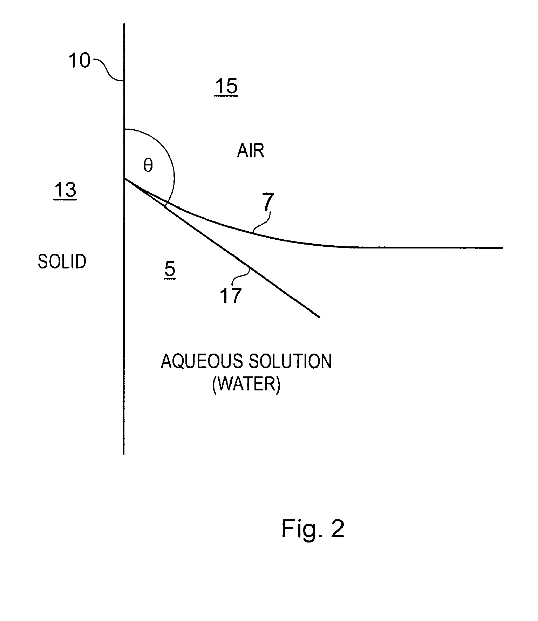

Figure 2 illustrates an example meniscus 7 in a well plate. The shape of the meniscus can be explained by standard fluid mechanics. Namely, the situation that arises in a well plate is a specific example of contact at a surface (well plate side wall 10) between a solid (well plate material 13) and two immiscible fluids (air 15 and water 5). Here we describe the aqueous solution that is contained in the well plate as water for simplicity. Similarly, the ambient gaseous fluid is described as air, although other gases may be used, for example helium, nitrogen or another inert gas or gas mixture. Moreover, reference to aqueous solution does not exclude the possibility that constituents other than water are in the liquid in solution or otherwise. For example, a solvent may be present for killing cells or other reasons.

Surface tension forces arise due to the energy needed to form an interface. At the point where the air-water interface meets the solid, a contact angle θ is defined by the tangent 17 to the air-water interface. When the surface tension forces are balanced the contact angle is defined by Young's Equation which for the example here of an air, water, solid system reads:

$$\gamma_{water\text{-}solid} - \gamma_{air\text{-}solid} = \gamma_{air\text{-}water} \cos \theta$$

where $\gamma_{air\text{-}water}$ is the surface tension between the two fluids air and water, $\gamma_{air\text{-}solid}$ is the surface tension between air and the solid, and $\gamma_{water\text{-}solid}$ is the surface tension between water and the solid. In the literature, the contact angle defined by Young's equation is often referred to as the static contact angle to differentiate it from the more complex situation of dynamic flow, as occurs in a capillary for example, where there is a dynamic contact angle.

The (static) contact angle is thus the standard parameter used to describe meniscus formation. In the figure, the illustrated meniscus is concave, which is generally associated with a hydrophilic surface. However, for some systems the meniscus is convex (i.e. bends down rather than up as viewed in the illustration), which is generally associated with a hydrophobic surface.

The terms hydrophilic and hydrophobic are used in the art generically for all fluids, not specifically to describe situations where water or aqueous solutions are involved. Some authors define the boundary between hydrophilic and hydrophobic surfaces rigorously as a contact angle of 90 degrees. More usually, a hydrophilic surface is regarded as one with good wettability, perhaps with a contact angle of less than 30-40 degrees, and a hydrophobic surface is regarded as one with poor wettability, perhaps with a contact angle in excess of 60-80 degrees. Surfaces with contact angles of approximately 90 degrees are often referred to as hydrophobic in the literature.

In this document we do not follow an exact definition of the terms hydrophilic and hydrophobic, while nevertheless following predominant standard usage that: a hydrophilic surface has a contact angle in the range from a few degrees to 30-40 degrees; a hydrophobic surface has a contact angle of greater than 60-80 degrees, often in the range 80-100 degrees; and a strongly hydrophobic surface is a hydrophobic surface with a contact angle greater than 90 degrees, typically greater than 100 degrees.

Having described the underlying physical description of meniscus formation, an embodiment of the invention is now described.

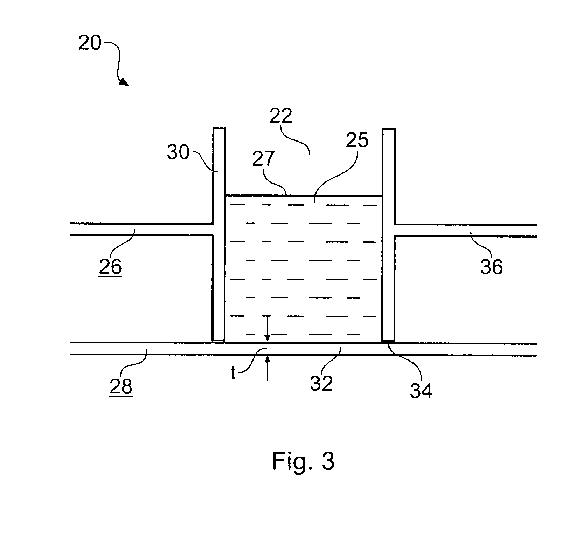

Figure 3 schematically illustrates in side section view a part of a well plate 20 embodying the invention, the part showing a single well 22 of the well plate 20. The well is shown partially filled with a liquid 25. The well plate is formed in two pieces, namely a first part 26, referred to as the body part in the following, and a second part 28, referred to as the base part in the following. As is conventional, the well 22 is defined by a side wall 30 and a base 32. However, in the current design the side wall 30 and the base 32 are made from separate parts, namely the body part 26 and base part 28 respectively which form a water-tight junction 34.

The body part 26 has tubular structures that form the cylindrical side walls 30 of the wells 22, where the tubes are interconnected by webs 36 that laterally extend between adjacent side walls. The webs 36 may be formed midway up the side walls, or at another position, for example at the top to provide an upper surface flush with the top of the wells. Multiple web layers may be provided if desired.

The base part 28 is formed as a single featureless transparent sheet of constant thickness 't'. By forming preferably all the well bases, but at least multiple well bases, from a single sheet, the base thickness can be made low, for example less than about 400 microns, and can be as thin as 20 to 100 micrometers and can exhibit superior imaging optical properties compared to existing well plates. Zeonor (TM) which is a transparent cyclo-olefin polymer (COP) manufactured by Zeon Corporation is a preferred base material. Zeonor has >85% transmission from 450-800 nm. With Zeonor base material, the imaging under liquid optical properties of the well plates of the present invention can approach those of optical glass. The well plate is particularly useful for measuring fluorescent events that can take place within the wells. The combination of the materials for the well wall and the thin bottom material, provides for particularly low fluorescent background.

The well bases 32 are then constituted by circular shaped portions of the base part 28 defined solely by the side-wall defining tubes of the body part 26.

The liquid 25 is shown partially filling the well 22 to define a water-air interface 27. The water-air interface 27 is shown schematically as being completely horizontal over its full lateral extent so that where it meets the side wall 30 there is no meniscus. In other words, with reference to Figure 2 and its description, the contact angle is illustrated as being exactly 90 degrees. In practice, it is merely desired that the contact angle is sufficiently close to 90 degrees that the meniscus formation is not large enough in terms of height and width to disrupt the functionality of the well plate. The contact angle is selected having regard to the surface energy, i.e. the degree of hydro-phobicity and -philicity, of the material used to construct the body part 26, more specifically, the degree of hydro-phobicity and -philicity of the inner surface of the side walls 30 after manufacture which may or may not include surface treatments or coatings to adjust the degree of hydro-phobicity and -philicity of the relevant surfaces.

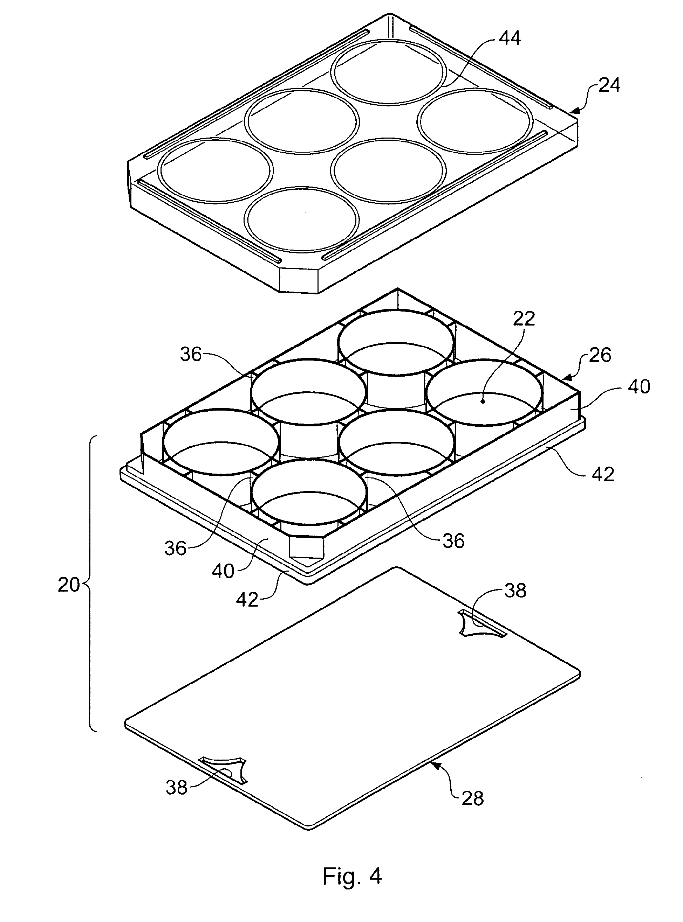

Figure 4 is a perspective exploded view of a two-part well plate 20 embodying the invention. In the exploded figure, the base part 28 is shown disassembled from the body part 26. A lid 24 for the well plate is also shown. The figure shows a 6-well well plate by way of example.

The base part 28 is made of suitable transparent material, for example a COP such as Zeonor (TM), but possibly a polymethyl methacrylate (PMMA) material or some other polymeric material or a glass. The transparent base material is in any case selected having regard to its optical properties. Preparation of the base part 28 will in most cases include applying an appropriate surface treatment or surface coating to render it sufficiently hydrophilic to support cell adherence, e.g. to make its contact angle 10-30 degrees. However, if the base part is made of a material which is sufficiently naturally hydrophilic, or cell adherence is not important for the intended use of the well plate, then that will not be necessary.

The base part 28 is a substantially featureless generally rectangular sheet of constant thickness, although there are two small holes 38 at either end away from the well areas to assist assembly. The base part is fused to the body part with an ultrasonic bonding process. Other forms of bonding may be used depending on the materials. For example, if the base part is glass, then a contact adhesive may be used.

The body part 26 is made of a black material, such as Zeonor (TM) or another COP which naturally has a contact angle close to 90 degrees, so the conventional plasma or other treatment to render the surface hydrophilic is foregone to retain a hydrophobic surface. The untreated natural COP surface thus does not support a meniscus, or in reality only supports a small convex or small concave meniscus. The body part 26 has a regular 3 x 2 square grid array of six wells 22 interconnected and supported by its webs 36. A standard well plate outer form is provided by outer walls 40 and a lower skirt 42 which are also part of the body part 26 and connected to the webs 36.

It is convenient if the base part and the body part are made of the same substances, e.g. Zeonor, but are distinguished by their different surface energies. In particular, the combination of an untreated body part and a treated base part is highly convenient for manufacture.

The lid 24 has the general form of a standard well plate lid, but includes circular lips 44 on the underside of the lid 24 to locate with the tops of the well side walls. This may be done by giving the lips 44 an inner diameter slightly larger than the outer diameter of the tubular side walls 30.

The plasma treatment process for modifying the hydrophobicity of the base and/or wall parts of the wells is now described. Plasma treatment of a variety of plastics materials used for biological sample containers, such as well plates and Petri dishes, is well known. The plasma treatment is typically used to render the container surface hydrophilic (i.e. improved wettability) using oxygen or other oxidizing gases such as air, water (in vapor), or nitrous oxide.

As previously mentioned the principal materials used for commercially available well plates to date are PS and PP. Plasma treatment processes to render PS hydrophilic are well known [3, 4, 5, 6] as are plasma treatment processes to render PP hydrophilic [7, 12]. Moreover, plasma treatments for PMMA [3], PE [5, 12], polyamide (nylon) [12], polycarbonate (PC) [12] and PI [7, 12] are also known. Further, contact angles of various COPs is known [8, 9, 10, 11], in some cases also after UV treatment [10]. COPs include Zeonor (TM) and Zeonex (TM) from Zeon Corporation, Topas (TM) from Ticona, Arton (TM) from Japan Synthetic Rubber Corporation and APEL (TM) from Mitsui Chemical Corporation. The natural (pre-treatment) contact angles of a variety of materials to a water/air fluid combination is tabulated below together with the minimum contact angle achieved with plasma treatment or UV treatment, with literature references for the tabulated values indicated in superscript.

**Table 1**

| CONTACT ANGLE (DEGREES) | Natural | Treated |
|---|---|---|
| | | |
| Polystyrene (PS)[3] | 87 | 20 |
| Polystyrene (PS)[11] | 89 | 4-25 |
| Polypropylene (PP)[12] | 87 | 22 |
| Polyethylene (PE)[12] | 87 | 22 |
| Cycloolefin (co-)polymer (COP) Zeon Zeonex 480R[11] | 92 | 4-36 |
| Cycloolefin (co-)polymer (COP) Zeon Zeonor 1020R (TM)[10] | 91 | 14 |
| Cycloolefin (co-)polymer (COP) Ticona Topas (TM)[11] | 97 | 4-29 |
| Cycloolefin (co-)polymer (COP) Ticona Topas (TM)[9] | 116 | n/a |
| Cycloolefin (co-)polymer (COP) Mitsui APEL (TM)[9] | 100-103 | n/a |
| Cycloolefin (co-)polymer (COP) Japan Synthetic Rubber Arton (TM)[9] | 97 | n/a |
| Styrene-acrylonitrile copolymer (SAN) BASF Luran (TM)[11] | 79 | 2-27 |
| Polyamide (nylon) [12] | 73 | 15 |
| Polyimide (PI)[12] | 79 | 10 |
| Polycarbonate (PC)[12] | 75 | 33 |
| Perspex (PMMA)[3] | 67 | 38 |
| Polymethyl Methacrylate (PMMA) Röhm VQ105[11] | 60 | 4-53 |

Any of these materials may be used for the well bases and/or side walls, either in surface treated or untreated (natural) form, to provide the desired combination of properties for well plates embodying the invention, as well as any of the materials and surface modification processes referred to in references [3-12] the relevant parts for which are incorporated herein by reference.

In all cases, the hydrophilicity is controllable in the process in that the contact angle gradually reduces from the natural level as a function of processing time. For example, assuming a typical gaseous ion density of say $10^{10}$ - $10^{12}$ cm$^{-3}$, e.g. oxygen density, the contact angle will gradually reduce from the natural level to the minimum achievable processing times over a period of around 5-10 minutes. If the plasma reactor is RF assisted, the speed of the contact angle reduction will also scale with microwave power.

In the context of the invention, such surface treatments may be used to reduce the hydrophobicity of the body part from say 116 degrees (the table value for Topas (TM)) to close to 90 degrees. Moreover, surface treatment may be used to reduce the hydrophobicity of the base part from any level to 10-40 degrees or less.

Plasma processes have also been developed to make surfaces hydrophobic (i.e. reduced wettability), for example many polymers can be rendered hydrophobic using fluorinated gases, such as yetrofluoromethane (CF4), sulfur hexafluorine (SF6), and perfluorohydrocarbons.

In the context of the invention, such surface treatments may be used to increase the hydrophobicity of the body part so that it attains close to 90 degrees.

It is noted that the wide variety of known combinations of materials and plasma treatment processes have varying degrees of long-term stability, so the choice of treatment for well plates will be influenced by this, with the more highly stable treatments with a shelf life of a year or more being preferred.

Any of the above-mentioned known processes and other known processes for modifying wettability may be applied to manufacture well plates according to the invention, either to the well base material, well wall material, or both.

Well plates of the invention are of particular interest for automated cell counting and confluence detection methods as described in our co-pending patent application US 11/050,826, the full contents of which are incorporated herein by reference. For culturing in a well plate, confluence is the degree to which the cells have grown to fill the well or other biological sample container. One speaks of a well being 70% confluent, 80% confluent and so forth, where 100% confluence is when the cells all touch each other filling the plate. The term subconfluent is also used to refer to a plate in which the cell colony or other cell aggregate has not yet reached confluence. For many applications, full confluence must be avoided, since it is associated with cell death or other undesired activity. Typically one aims to replate when confluence reaches a threshold, such as 70%. We propose an automated cell counting and/or confluence detection method based on phase contrast microscopy in which the automated method is carried out using a well plate of the invention in combination with image capture with phase contrast microscopy followed by application of a suitable image processing algorithm.

The degree of confluence can be determined by an automated cell count which is translated into an area by multiplication of the cell count by an area representing an average area for the cell type being cultured. Alternatively, the degree of confluence is determined by processing the image to: establish cell boundaries, compute the area of each cell from the cell boundary, and sum the cell areas. Image processing software, or alternatively any mixture of software, firmware and hardware, can be used to perform the image processing.

The cell boundaries can be determined directly by contrast from the plasma membrane, or from the extent of the cytoplasm, or possibly in some cases from contrast provided by an extracellular fluid in which the cell is located. The method may be carried out with no fluorescence staining in many cell types of interest, but if desired or necessary to achieve sufficient contrast modifying the cells by inclusion of a fluorescent tag may be performed, e.g. to image other cell parts, such as the nucleus, or to assess the physiological state of the cell, such as cell cycle. For example, red lectin can be used to tag the cell membranes. Nuclear tags that do not kill the cells may also be suitable to provide contrast in the case that the aggregate area of the cells is determined by cell counting rather than by direct cell area calculation. Whole cell stains may also be considered, such as Phalloidin FM4-64. A variety of suitable dyes are known and can be selected, for example, from the Molecular Probes catalog.

To determine confluence for a given well, the process is as follows. The captured image of the cells adjacent the base of the well is divided into background and foreground by extracting the background. This is done by applying a large Gaussian blur filter to the image, then subtracting this from the original image before adding the mean of the original image to each pixel. After this operation, pixels with intensities close to the mean of the resulting image are considered background, the remainder are considered foreground. The closeness to the mean is adjustable to accommodate variations in lighting etc. A segmented binary image is then generated by assigning foreground pixels to white and background pixels to black.

We envisage measuring the degree of confluence in one of two ways.

The first way involves counting the cells, and then assuming a value for cell area. This is usually reliable, since the variance in average cell area of a given cell type is usually small. The degree of confluence is then calculated to by the number of cells multiplied by the cell area divided by the available area of the well or other substrate, plate or dish.

The second way is to directly measure the aggregate area of all the cells by image processing of each individual cell to determine its boundary and thus area. The area of the cells can then be scaled up by a packing factor, e.g. assuming hexagonal close packing, before being divided by the available area of the well to arrive at a degree of confluence.

It will be appreciated that an automated cell counting process is a special simplified case of the confluence determination process in which cell areas (and thus boundaries) are not generally relevant.

By using a well plate according to the invention confluence detection and/or cell counting processes are much less demanding to automate, since both the imaging and the image processing are made easier and quicker. Imaging is easier and quicker since cell growth on the well side walls can be inhibited thereby ensuring that the cells lie in a single common plane defined by the base. Image processing is easier and quicker, since the cells lie in a common plane, so the image processing task can be reliably assumed to be a two-dimensional problem. For example, multiple images taken at vertically offset focal planes are not required, since it can be reliably assumed that no cells have adhered to the (hydrophobic) side walls. In addition, the two-part well plate construction allows superior optical performance to be achieved, for the reasons described above, so the quality of the images acquired for the image processing will be high across the full area of the base. This will also improve the accuracy of coordinate determination of the cells, which will in turn improve the reliability of picking that may follow from replating decisions made on the basis of the confluence having reached a predetermined threshold. These processes may be carried out on a Genetix CloneSelect (TM) Imager, ClonePix (TM) or ClonePix FL (TM) products, for example.

Finally, although confluence detection and cell counting processes are described here primarily in terms of automated processes, the advantages gained by use of well plates according to the invention will also benefit manual or semi-automated confluence detection and cell counting processes, e.g. a user viewing through a microscope will see a clearer image with no black meniscus-induced ring and no optical distortion in the region of the base-wall junction.

In summary, applying the invention, it is possible to provide well plates with unique combinations of desirable properties. In particular, meniscus formation can be suppressed by suitable choice of the surface energy of the well side walls. Moreover, the well bases can be designed with superior optical properties made possible by forming the well bases from a different material than the side walls, and also by making the well bases from a separate part (or parts), which in particular allows the well bases to be made very thin while retaining sufficient structural integrity. Still further, regardless of whether optical properties are of interest, the separate formation of the well bases and side walls allows the degree of hydrophilicity and hydrophobicity or the well and base to be selected freely. In particular, the combination of a relatively hydrophilic bases and hydrophobic wells promotes cell growth on the bases and not the side walls. Using such well plates it is less demanding to obtain good data when applying existing protocols, such as the examples 1-8 of reference [1] which are incorporated herein by reference in their entirety. Moreover, use of plates with higher well density and smaller well volume becomes possible leading to savings in reagent volumes and increases in the degree of automation.

## REFERENCES

[1] US 2004/0202582 A1 (Coassin et al)

[2] H. Raabe, G. Moyer, G. Mun, M. Clear, and J.W. Harbell "INDUCTION OF A ZONE OF CELL DEATH IN MULTI-WELL PLATES BY REFEEDING" Society of Toxicology, 2004, Baltimore, MD

[3] TA Baede, REJ Sladek, E. Stoffels "Surface Modification of substrates for bacteria and cell culture" XXVIIth ICPIG, Eindhoven, the Netherlands, 18-22 July 2005

[4] S. Guruvenket, Manoj Komath, S. P. Vijayalakshmi, A. M. Raichur and G. Mohan Rao, 2003, "Wettability enhancement of polystyrene using electron cyclotron resonance plasma with argon," Journal of Applied Polymer Science, volume 90, pages 1618-1623 (2003)

[5] S. Guruvenket, G. Mohan Rao, Manoj Komath and AM Raichur "Plasma surface modification of polystyrene and polyethylene," Applied Surface Science, volume 236 pages 278-284 (2004).

[6] Callen, B.W., Ridge, M.L., Lahooti, S., Neumann, A.W., and Sodhi, R.N.S., "Remote Plasma and UV-Ozone Modification of Polystyrene", J. Vac. Sci. Technol. A 13, pages 2023-2029 (1995)

[7] Les Wood and James Getty "PLASMA PROCESSING FOR OPTIMAL MEDICAL DEVICE MANUFACTURING" http:// www.marchplasma.com/ pdf/March_SMTA_2005.pdf

[8] P. Mela, A. van den Berg, Y. Fintschenko, EB Cummings, BA Simmons, BJ Kirby "The zeta potential of cyclo-olefin polymer microchannels..." Electrophoresis, volume 26, pages 1792-1799 (2005)

[9] JY Shin, JY Park, C. Liu, J. He, SC Kim, "Chemical Structure and Physical Properties of Cyclic Olefin Copolymers", Pure Appl. Chem. 77(5), 801-814, 2005

[10] C Li, Y Yang, HG Craighead and KH Lee "Isoelectric Focusing in Cyclic Olefin Copolymer Microfluidic Channels Coated by Polyacrylamide Using a UV Photografing Method" Electrophoresis, volume 26, pages 1800-1806 (2005).

[11] BL Johansson, A. Larsson, A. Ocklind, and A. Ohrlund "Characterization of Air Plasma-Treated Polymer Surfaces by ESCA and Contact Angle Measurements for Optimization of Surface Stability and Cell Growth" J. Appl. Polymer Sci., volume 86, pages 2618-2625 (2002)

[12] Anatech Limited website article "Plasma Surface Treatment for Life Science Applications" http://www.anatechltd.com/plasma surface treatment

[13] US 2004/0020595 A1 (Khan et al)

[14] US 2005/0173059 A1 (Ringleben et al)

## Claims

1. A well plate (20) having at least one well (22), each well being defined by a side wall (30) and a base (32), **characterised in that** the side wall has a surface energy that provides a contact angle of approximately 90 degrees for an aqueous solution (25) contained in the well in an ambient environment, thereby to suppress meniscus formation.

**2.** The well plate of claim 1, wherein the base has a surface energy that is lower than the side wall surface energy to promote preferential attachment of cells to the base relative to the side wall.

**3.** The well plate of claim 1, wherein the side wall is made of a material that naturally has a contact angle of approximately 90 degrees.

**4.** The well plate of claim 1, wherein the side wall is made of a material from one of the group consisting of: cycloolefin polymer, polyethylene, polypropylene, polymethyl methacrylate, styrene-acrylonitrile copolymer, polyamide, polyimide, and polycarbonate.

**5.** The well plate of claim 1, wherein the side wall is made of polystyrene.

**6.** The well plate of claim 1, wherein the base has a surface energy that provides a contact angle of between any one of 2, 4, 10, 15, 20 or 30 degrees and any one of 25, 30, 35 or 50 degrees, in particular between 15 and 25 degrees, for an aqueous solution (water) contained in the well in an ambient (air) environment.

**7.** The well plate of claim 1, wherein the base is made of a material from one of the group consisting of: cycloolefin polymer, polyethylene, polypropylene, polymethyl methacrylate, styrene-acrylonitrile copolymer, polyamide, polyimide and polycarbonate.

**8.** The well plate of claim 1, wherein the base is made of polystyrene.

**9.** The well plate of claim 1, wherein the base is made of glass.

**10.** The well plate of claim 1, wherein at least the base is made of transparent material.

**11.** The well plate of claim 10, wherein at least the base is made of a low-fluorescence material having less than 80% of the fluorescence per unit volume of polystyrene.

**12.** A method of manufacturing a well plate (20) having at least one well (22), each well being defined by a side wall (30) and a base (32), the method comprising:

   providing a first part (26) defining the side wall of each well, wherein the side walls have a contact angle of approximately 90 degrees;
   providing a second part (28) defining the base of each wall; and
   connecting the first and second parts so that the base of each well forms a water-tight seal with its associated side wall.

**13.** The method of claim 12, wherein the first part is made of a material that has a contact angle of approximately 90 degrees by virtue of its natural properties.

**14.** The method of claim 13, wherein the first part is made of polystyrene and the second part is made of glass.

**15.** The method of claim 12, further comprising: treating the first part to modify its surface energy to provide a contact angle of approximately 90 degrees for a water-air interface abutting the side wall.

**16.** The method of claim 15, wherein the first part is made of a material that is naturally hydrophobic with a contact angle of greater than 90 degrees and said treating increases its surface energy until the contact angle is reduced to approximately 90 degrees.

**17.** The method of claim 15 or 16, wherein said treating is a plasma process.

**18.** The method of claim 12, wherein the first and second parts are connected by fusing them together with an ultrasonic bonding process.

**19.** The method of claim 12, wherein the first and second parts are connected by adhesive bonding.

**20.** The method of claim 12, wherein the first part is made of polystyrene and the second part is made of polystyrene.

21. The method of any one of claims 12 to 20, further comprising: treating the second part to modify its surface energy to make it lower than that of the first part to promote preferential attachment of cells to the base relative to the side wall.

22. A method comprising:

providing a well plate (20) having at least one well (22), each well being defined by a side wall (30) formed by a first part (26) of the well plate and a base (32) formed by a second part (28) of the well plate, wherein for each well the base has a surface energy that is lower than the surface energy of the side wall; and
growing cells in aqueous solution (25) in each well, wherein the cells preferentially attach to the base owing to the lower surface energy of the base relative to the side wall.

23. The method of claim 22, further comprising: imaging the well plate from above or below to view the cells.

24. The method of claim 23, further comprising: digitally storing an image of a plane at or adjacent to the base; and processing the image to count the cells.

25. The method of claim 23, further comprising: digitally storing an image of a plane at or adjacent to the base; and processing the image to determine a degree of confluence of the cells.

26. The method of claim 22, wherein the cells are individual cells.

27. The method of claim 22, wherein the cells form a plurality of colonies.

28. A well plate (20) of non-unitary construction having at least one well (22), each well being defined by a side wall (30) and a base (32), wherein the well plate is constructed from a first part (26) defining the side wall of each well and a second part (28) defining the base of each wall, the first and second parts having respective first and second surface energies, **characterised in that** the second surface energy for the base of each well is lower than the first surface energy to promote preferential attachment of cells to the base relative to the side wall.

29. The well plate of claim 28, wherein the second part is transparent.

30. The well plate of claim 28, wherein the first part is made of polystyrene and the second part is made of polystyrene.

31. The well plate of claim 28, wherein the first part is made of polystyrene and the second part is made of glass.

32. A well plate (20) of non-unitary construction having at least one well (22), each well being defined by a side wall (30) and a base (32), wherein the well plate is constructed from a first part (26) defining the side wall of each well and a second part (28) defining the base of each wall, the first and second parts having respective first and second surface energies, **characterised in that** the second surface energy for the base of each well is higher than the first surface energy to promote cell growth of cells adhered to the base relative to cells adhered to the side wall.

33. The well plate of claim 32, wherein the second part is transparent.

34. The well plate of claim 32, wherein the first part is made of polystyrene and the second part is made of polystyrene.

35. The well plate of claim 32, wherein the first part is made of polystyrene and the second part is made of glass.

ELP 1 859 866 A1

Fig. 1A

Fig. 1B

13

10

15

13

θ

AIR

7

SOLID

5

17

AQUEOUS SOLUTION
(WATER)

Fig. 2

Fig. 3

Fig. 4

EP 1 859 866 A1

**European Patent Office**

**EUROPEAN SEARCH REPORT**

Application Number

EP 07 25 0717

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 648 536 A1 (EIDGENOESS MUNITIONSFAB THUN [CH]) 19 April 1995 (1995-04-19)<br><br>* column 1, line 5 - line 45 *<br>* column 2, line 46 - column 4, line 15 *<br>----- | 1-4,6-8, 10-13, 15,28,29 | INV.<br>B01L3/00<br>C12N5/00 |
| X | WO 03/050515 A (ARTEL INC [US]; CURTIS RICHARD H [US]) 19 June 2003 (2003-06-19)<br>* page 5, paragraph 2 *<br>* page 8 - page 10; figures 3A,4 *<br>----- | 1-8,10, 11 | |
| X | US 2005/121782 A1 (NAKAMURA KOICHIRO [JP] ET AL) 9 June 2005 (2005-06-09)<br><br>* the whole document *<br>----- | 1-4,6, 9-11,28, 29 | |
| X | EP 0 290 125 A (MOLECULAR DEVICES CORP [US]) 9 November 1988 (1988-11-09)<br><br>* the whole document *<br>----- | 1-8, 10-13, 15,20,21 | |
| X | US 2005/170498 A1 (DOLLEY PAULA J [US] ET AL) 4 August 2005 (2005-08-04)<br><br><br>* paragraph [0030] - paragraph [0042]; figure 4 *<br>----- | 12-14, 16,19, 22,23, 26-29,31 | TECHNICAL FIELDS SEARCHED (IPC)<br><br>B01L<br>C12N<br>C12M |
| X | EP 1 188 481 A (AGFA GEVAERT [BE]) 20 March 2002 (2002-03-20)<br>* paragraph [0016] - paragraph [0018] *<br>* paragraph [0022] *<br>* paragraph [0028] *<br>* paragraph [0032] - paragraph [0033] *<br>* paragraph [0043] *<br>* paragraph [0054] *<br>-----<br><br>-/-- | 28-35 | |

The present search report has been drawn up for all claims

| Place of search | Date of completion of the search | Examiner |
|---|---|---|
| Munich | 28 August 2007 | Marti, Pedro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

17

European Patent
Office

**EUROPEAN SEARCH REPORT**

Application Number

EP 07 25 0717

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | US 2004/202582 A1 (COASSIN PETER J [US] ET AL) 14 October 2004 (2004-10-14)<br><br>* paragraph [0074] - paragraph [0118] *<br>* paragraph [0140] - paragraph [0141] *<br>----- | 12,14, 18,20, 22-31 | |
| A | US 2005/279730 A1 (MIYAKE HIDEYUKI [JP] ET AL) 22 December 2005 (2005-12-22)<br>* the whole document *<br>----- | 1-35 | |
| | | | TECHNICAL FIELDS SEARCHED (IPC) |

The present search report has been drawn up for all claims

| Place of search | Date of completion of the search | Examiner |
|---|---|---|
| Munich | 28 August 2007 | Marti, Pedro |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P04C01)

European Patent
Office

Application Number

EP 07 25 0717

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**European Patent**
**Office**

**LACK OF UNITY OF INVENTION**
**SHEET B**

Application Number

EP 07 25 0717

The Search Division considers that the present European patent application does not comply with the
requirements of unity of invention and relates to several inventions or groups of inventions, namely:

1. claims: 1-21

    Claims 1-11: Well plate characterised in that the side wall
    has a surface energy that provides a contact angle of
    approximately 90° for an aqueous solution, thereby to
    suppress meniscus formation.
    Claims 22-21: Method of manufacturing a well plate
    comprising a first part defining the side wall of each well,
    wherein the side wall have a contact angle of 90°.
    ---

2. claims: 22-31

    Claims 22-27: Method for growing cells in a well plate,
    wherein for each well the base has a surface energy that is
    lower than the surface energy of the side wall.
    Claims 28-31: Well plate of non-unitary construction
    characterised in that the surface energy for the base is
    lower than the surface energy for the side wall to promote
    preferential attachment of cells to the base relative to the
    side wall.
    ---

3. claims: 32-35

    Well plate of non-unitary construction characterised in that
    the surface energy for the base of each well is higher than
    the surface energy for the side wall to promote growth of
    cells adhered to the base relative to cells adhered to the
    side wall.
    .

    .
    The common inventive concept linking together all groups is
    a well plate having side walls and a base. Since this
    concept is not novel in view of the cited prior art, the
    different subject-matters are not so linked as to form a
    single general inventive concept. Moreover, group A, on one
    side, and groups B and C, on the other side, solve different
    technical problems. Therefore, the claims lack unity within
    the meaning of Art. 82 EPC.
    ---

**ANNEX TO THE EUROPEAN SEARCH REPORT
ON EUROPEAN PATENT APPLICATION NO.**

EP 07 25 0717

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2007

| Patent document cited in search report | | Publication date | Patent family member(s) | | | Publication date |
|---|---|---|---|---|---|---|
| EP 0648536 | A1 | 19-04-1995 | AT | 142908 | T | 15-10-1996 |
| | | | CH | 687592 | A5 | 15-01-1997 |
| | | | DE | 59400685 | D1 | 24-10-1996 |
| | | | DK | 648536 | T3 | 17-03-1997 |
| | | | JP | 7190924 | A | 28-07-1995 |
| | | | US | 5540891 | A | 30-07-1996 |
| WO 03050515 | A | 19-06-2003 | AU | 2002350240 | A1 | 23-06-2003 |
| | | | DE | 02786771 | T1 | 10-02-2005 |
| | | | EP | 1454125 | A2 | 08-09-2004 |
| | | | JP | 2005512085 | T | 28-04-2005 |
| | | | US | 2003107738 | A1 | 12-06-2003 |
| | | | US | 2004246501 | A1 | 09-12-2004 |
| US 2005121782 | A1 | 09-06-2005 | NONE | | | |
| EP 0290125 | A | 09-11-1988 | US | 4741619 | A | 03-05-1988 |
| US 2005170498 | A1 | 04-08-2005 | NONE | | | |
| EP 1188481 | A | 20-03-2002 | NONE | | | |
| US 2004202582 | A1 | 14-10-2004 | NONE | | | |
| US 2005279730 | A1 | 22-12-2005 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 11050826 B
- US 20040202582 A1, Coassin
- US 20040020595 A1, Khan
- US 20050173059 A1, Ringleben

### Non-patent literature cited in the description

- **H. RAABE ; G. MOYER ; G. MUN ; M. CLEAR ; J.W. HARBELL.** INDUCTION OF A ZONE OF CELL DEATH IN MULTI-WELL PLATES BY REFEEDING. Society of Toxicology, 2004
- Surface Modification of substrates for bacteria and cell culture. **TA BAEDE ; REJ SLADEK ; E. STOFFELS.** XXVIIth ICPIG. 18 July 2005
- **S. GURUVENKET ; MANOJ KOMATH ; S. P. VIJAYALAKSHMI ; A. M. RAICHUR ; G. MOHAN RAO.** Wettability enhancement of polystyrene using electron cyclotron resonance plasma with argon. *Journal of Applied Polymer Science,* 2003, vol. 90, 1618-1623
- **S. GURUVENKET ; G. MOHAN RAO ; MANOJ KOMATH ; AM RAICHUR.** Plasma surface modification of polystyrene and polyethylene. *Applied Surface Science,* 2004, vol. 236, 278-284
- **CALLEN, B.W ; RIDGE, M.L ; LAHOOTI, S ; NEUMANN, A.W ; SODHI, R.N.S.** Remote Plasma and UV-Ozone Modification of Polystyrene. *J. Vac. Sci. Technol. A,* 1995, vol. 13, 2023-2029
- **LES WOOD ; JAMES GETTY.** *PLASMA PROCESSING FOR OPTIMAL MEDICAL DEVICE MANUFACTURING,* http:// www.marchplasma.com/ pdf/March_SMTA_2005.pdf
- **P. MELA ; A. VAN DEN BERG ; Y. FINTSCHENKO ; EB CUMMINGS ; BA SIMMONS ; BJ KIRBY.** The zeta potential of cyclo-olefin polymer microchannels. *Electrophoresis,* 2005, vol. 26, 1792-1799
- **JY SHIN ; JY PARK ; C. LIU ; J. HE ; SC KIM.** Chemical Structure and Physical Properties of Cyclic Olefin Copolymers. *Pure Appl. Chem.,* 2005, vol. 77 (5), 801-814
- **C LI ; Y YANG ; HG CRAIGHEAD ; KH LEE.** Isoelectric Focusing in Cyclic Olefin Copolymer Microfluidic Channels Coated by Polyacrylamide Using a UV Photografing Method. *Electrophoresis,* 2005, vol. 26, 1800-1806
- **BL JOHANSSON ; A. LARSSON ; A. OCKLIND ; A. OHRLUND.** Characterization of Air Plasma-Treated Polymer Surfaces by ESCA and Contact Angle Measurements for Optimization of Surface Stability and Cell Growth. *J. Appl. Polymer Sci.,* 2002, vol. 86, 2618-2625
- *Plasma Surface Treatment for Life Science Applications, http://www.anatechltd.com/plasma surface treatment*